# EUROPÄISCHE PATENTSCHRIFT

(11) **EP 0 673 915 B1**
(45) Veröffentlichungstag und Bekanntmachung des Hinweises auf die Patenterteilung: **18.11.1998**
(21) Anmeldenummer: 95810131.3
(22) Anmeldetag: 02.03.1995
(51) Int. Cl.: C07C 69/732

(54) **Verfahren zur Herstellung von Hydroxyphenylcarbonsäureestern**
Process for the preparation of hydroxyphenylcarboxylic acids
Procédé de préparation d'acides hydroxyphénylcarboxyliques

(30) Priorität: 11.03.1994 CH 727/94
(43) Veröffentlichungstag der Anmeldung: 27.09.1995
(73) Patentinhaber: Ciba Specialty Chemicals Holding Inc., 4057 Basel (CH)
(72) Erfinder: Kleiner, Christoph, Dr., I-40037 Sasso Marconi (IT)

(56) Entgegenhaltungen:
- EP-A- 0 300 055
- EP-A- 0 300 056
- EP-A- 0 437 187

## Beschreibung

Die Erfindung betrifft ein Verfahren zur Herstellung von Hydroxphenylcarbonsäureestern sowie die Verwendung des eingesetzten Katalysators.

Die unten beschriebenen und ähnliche Hydroxyphenylcarbonsäureester der Formel I sind als Antioxidantien von Nutzen und können durch Umesterung nach einer Reihe bekannter Verfahren hergestellt werden.

In der Europäischen Patentanmeldung Nr. 437 187 wird die Herstellung von Hydroxyphenylcarbonsäureestern in Gegenwart von Alkalimetallsalzen als Katalysatoren mit Erdalkalimetalloxiden, -hydroxiden, -aluminaten, -oder -silicaten als Trägern beschrieben.

Da unter den Verbindungen der Formel I wichtige Handelsprodukte sind, besteht weiterhin ein Bedarf an neuen, verbesserten Verfahren zu ihrer Herstellung.

Überraschenderweise wurde nun gefunden, daß man bei Verwendung von Magnesiumacetat als Katalysator wesentlich geringere Mengen des Katalysators benötigt und Produkte erhält, die direkt eingesetzt werden können.

Die Erfindung betrifft somit ein Verfahren zur Herstellung von Verbindungen der Formel I 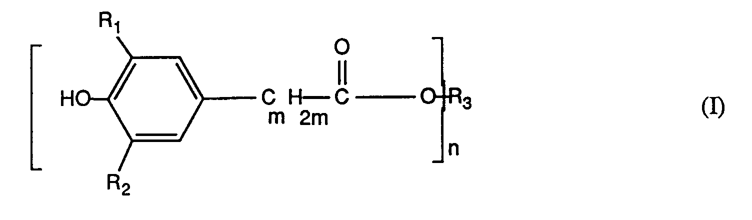
worin R₁ und R₂ unabhängig voneinander C₁-C₈-Alkyl oder Cyclopentyl oder -hexyl sind,
m 0, 1, 2, oder 3 ist,
n 1 oder 2 ist, und
R₃ je nach dem Wert von n C₄-C₂₀-Alkyl, C₅-C₁₂-Cycloalkyl, C₂-C₄-Alkylen oder durch Sauerstoff unterbrochenes C₄-C₁₂-Alkylen ist,
durch Umsetzung einer Verbindung der Formel II 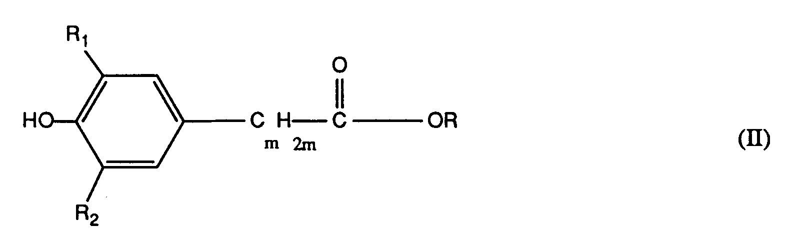
worin R für Methyl oder Ethyl steht,
mit einer Verbindung der Formel III

R₃(OH)ₙ (III)

dadurch gekennzeichnet, daß die Umsetzung in Gegenwart von Magnesiumacetat als Katalysator durchgeführt wird.

Stellen R₁ und R₂ C₁-C₈-Alkyl dar, so handelt es sich dabei um verzweigte oder unverzweigte Reste. Beispiele hierfür sind Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl und 2-Ethylhexyl. Vorzugsweise ist mindestens einer der beiden Reste R₁ und R₂ verzweigt. Für R₃ als C₄-C₂₀-Alkyl sind Beispiele der obigen Liste ab vier Kohlenstoffatomen zu entnehmen, zusätzlich kann R₃ noch z.B. Nonyl, Decyl, Undecyl, Dodecyl, Tridecyl, Tetradecyl, Pentadecyl, Hexadecyl, Heptadecyl, Octadecyl, Eicosyl, 1,1,3-Trimethylhexyl oder 1-Methylundecyl bedeuten.

Bevorzugt sind R₁ und R₂ Alkylreste mit 1-4 C-Atomen. Beispiele dafür sind obiger Liste zu entnehmen. Mindestens einer der beiden Reste R₁ und R₂ ist bevorzugt tert-Butyl.

R₃ als C₅-C₁₂-Cycloalkyl kann z.B. Cyclopentyl, Cyclohexyl, Cycloheptyl, Cyclooctyl oder Cyclododecyl sein. Cyclopentyl und Cyclohexyl sind bevorzugt, insbesondere Cyclohexyl.

Bevorzugt ist R₃ höheres Alkyl, z.B. C₈-C₂₀, besonders bevorzugt i-Octyl, 2-Ethylhexyl oder n-Octadecyl. Isooctyl steht für eine Mischung von Aklylresten, wie sie in der unter diesem Namen bekannten Alkoholmischung vorliegen (vgl. Merck Index, 10th Edition, Nr. 5041).

Im Falle n = 2 ist R₃ eine durch Weglassen der OH-Gruppen von einem zweiwertigen Alkohol wie etwa Ethylenglykol, Propylenglykol oder Butylenglykol abgeleitete Alkylengruppe, die durch Sauerstoff unterbrochen sein kann. Im letzteren Fall kann die Alkylengruppe bis 12 C-Atome enthalten. Bevorzugt sind jedoch 6 oder 4. Die Struktureinheit -CH₂-CH₂-O- findet sich darin bevorzugt. Beispiele sind die in der oben beschriebenen Weise von Diethylenglykol und Triethylenglykol abgeleiteten Gruppen.

Das Verfahren wird bevorzugt zur Herstellung von Verbindungen der Formel I angewandt, worin m für 2 steht.
Besonders bevorzugt werden Verbindungen der Formel I hergestellt, worin R₁ Methyl oder tert-Butyl, R₂ tert-Butyl und R₃ n-Octadecyl, i-Octyl, 2-Ethylhexyl oder einen vom Di- oder Triethylenglykol abgeleiteten Rest, -(CH₂CH₂O)ₐCH₂CH₂-, darstellen, wobei a für 1 oder 2 steht.
Ganz besonders bevorzugt werden Verbindungen der Formel I hergestellt, worin R₁ für Methyl oder tert-Butyl und R₂ für tert-Butyl stehen.

Die Erfindung betrifft auch die Verwendung von Magnesiumacetat als Katalysator bei der Herstellung von Verbindungen der Formel I durch Umsetzung von Verbindungen der Formel II mit Verbindungen der Formel III.

Ein besonderer Vorteil des erfindungsgemäßen Verfahrens liegt darin, daß das Produkt nach der Reaktion ungereinigt mit physikalischen Methoden in eine verwendungsfähige Handelsform gebracht werden kann. Dafür stehen-konventionelle Methoden wie Mahlen, Granulieren, Pelletieren oder Brikettieren zur Verfügung.

Das erfindungsgemäße Verfahren kann in einem inerten organischen Lösungsmittel durchgeführt werden, beispielsweise in aliphatischen oder aromatischen Kohlenwasserstoffen wie Octan, Decalin, Siedegrenzbenzinen bzw. Petroletherfraktionen oder Mischungen davon oder in Benzol, Toluol oder Xylol(en). Bevorzugt wird jedoch ohne Lösungsmittel gearbeitet.

Die Reaktionspartner der Formeln II und III werden zweckmäßig zur homogenen Schmelze gebracht, bevor der Katalysator zugegeben wird. Bevorzugt werden sie unter vermindertem Druck (beispielsweise zwischen 2 und 200 mbar, vorteilhaft bei 20 mbar) erhitzt, bis eine Schmelze vorliegt. Dies dient auch der Vortrocknung der Reaktionspartner. Empfehlenswerte Temperatur dafür ist z.B. 80-90°C.

Der Katalysator wird dem Reaktionsgemisch zweckmäßig in Mengen von 0,05 bis 3 mol%, bevorzugt von 0,05 bis 2 mol%, besonders bevorzugt 0,05 bis 0.3 mol%, bezogen auf die Verbindungen der Formel II, zugegeben.

Übliche Operationen wie Rühren des Reaktionsgemisches können von Vorteil sein.

Die Reaktionstemperatur liegt zweckmäßig bei 120 bis 210°C, bevorzugt bei 140 bis 200°C, besonders bevorzugt bei 160 bis 190°C.

Die Reaktionszeit kann in weiten Grenzen schwanken und liegt im allgemeinen je nach Druck und Temperatur zwischen einer und 12 Stunden und ist bevorzugt eine bis 10, insbesondere 2 bis 7 Stunden

Man kann die Reaktion ganz oder teilweise bei Normaldruck durchführen, aber zur Verschiebung des Gleichgewichts ist ein Unterdruck zweckmäßig. Arbeitet man mit Unterdruck, so beträgt der Druck zweckmäßig 1 bis 200 mbar, z.B. 1 bis 50, bevorzugt 1 bis 15 mbar. Da während der Reaktion Methanol frei wird, kann der Druck sich im Verlauf der Reaktion ändern. Z.B. steigt er in dem Mäße, wie Methanol freigesetzt wird. Ist dieses abgetrennt, so verringert man zweckmäßig den Druck weiter, vorzugsweise unter 2 mbar, bis die gegebenenfalls überschüssige Komponente III abgetrennt ist.

Bei Kristallisation direkt aus der Schmelze weist das Endprodukt einen vorteilhaft geringen Magnesiumgehalt auf, der bei der Verwendung als Stabilisator für gewöhnlich nicht stört.

Das Produkt der Formel I kann also entweder direkt durch Abkühlen und gegebenenfalls Impfen der Reaktionsschmelze zur Kristallisation gebracht werden, worauf es direkt zu einer Handelsform weiterverarbeitet werden kann, oder die Reaktionsschmelze wird in einem geeigneten Lösungsmittel aufgenommen, abgekühlt und gegebenenfalls unter Impfen kristallisiert. Als Lösungsmittel kommen in Frage: aliphatische Kohlenwasserstoffe wie Pentan, Hexan, Heptan, Octan, Cyclohexan, Decalin, Petrolether oder Mischungen davon; aromatische Kohlenwasserstoffe wie Benzol, Toluol oder Xylol; Alkohole und Alkohol/Wasser-Gemische wie Ethanol (80-100%), Methanol (80-100%) und Isopropanol (80-100%). Das Produkt kann vorteilhaft aus Methanol oder Isopropanol bzw. aus deren Gemischen mit Wasser umkristallisiert werden und wird dann sehr rein [im Falle von β-(3,5-Di-tert-butyl-4-hydroxyphenyl)propionsäurestearylester >99%, Smp. 50-52°C] erhalten.

In der Regel setzt man ungefähr äquivalente Mengen des Esters II und des Alkohols III ein. Zweckmäßig liegt das Verhältnis Reaktand II pro Äquivalent des Reaktanden III zwischen 0.8 : 1 und 1.4 : 1, vorteilhaft zwischen 0.85 : 1 und 1.3 : 1.

Wenn ein Überschuß an Ester II eingesetzt wird (vorteilhaft 5 bis 40 %, bevorzugt um 20 bis 30%), kann er als Schleppmittel zur Destillation unumgesetzten Alkohols und die Farbe des Produktes beeinträchtigender chinoider Nebenprodukte dienen.

Durch das vorliegende Verfahren ist direkt und ohne Reinigungschritte ein sehr reines Produkt zugänglich. Da sehr geringe Mengen an Katalysator benötigt werden, ist auch der Anteil des Katalysators im Produkt sehr klein. Da es sich um kein Schwermetall handelt, stört der Metallanteil für keine der bekannten Anwendungen. Besonders hervorzuheben ist die Tatsache, daß bei dem erfindungsgemäßen Verfahren Verfärbungen in der Reaktionsmasse und in den Produkten vermieden werden. Das Verfahren zeichnet sich ferner dadurch aus, daß ein Filtrationsschritt nicht vonnöten ist, und daß die Anzahl der Nebenkomponenten gering ist.

Die im erfindungsgemäßen Verfahren eingesetzten Verbindungen der Formeln II und III sind bekannt oder können nach an sich bekannten Verfahren hergestellt werden. In den eingangs zitierten Dokumenten sind Verbindungen der Formel II beschrieben.

Die erfindungsgemäß hergestellten Verbindungen der Formel I dienen beispielsweise dem Schutz von thermischem, oxidativem und/oder actinischem Abbau unterliegenden organischen Materialien wie z.B. Kunststoffen und Schmierstoffen und sind zum Teil im Handel erhältlich.

Die folgenden Beispiele erläutern die Erfindung weiter, ohne sie jedoch zu beschränken. Alle Angaben in Prozenten und Teilen beziehen sich auf das Gewicht, sofern nicht anders angegeben.

### Beispiel 1: β-(3,5-Di-tert-butyl-4-hydroxyphenyl)propionsäurestearylester (Verbindung der Formel I, worin R₁ und R₂ tert-Butyl sind, n = 1 und m = 2 sind, und R₃ für ⁿC₁₈H₃₇ steht)

202 g (0.69 mol) β-(3,5-Di-tert-butyl-4-hydroxyphenyl)propionsäuremethylester und 185 g (0.68 mol) Stearylalkohol (trocken) werden vorgelegt und bei 80°C und 20 mbar geschmolzen. Wenn alles geschmolzen ist, wird das Vakuum mit Stickstoff gebrochen, und 1 g (0.012 mol) Magnesiumacetat wird zugegeben. Es wird auf 185°C aufgeheizt, binnen einer Stunde auf 3 mbar evakuiert und 5 h bei 185°C gehalten. Die Schmelze enthält noch je ca. 1.5% der beiden Ausgangsstoffe. Die Reaktionsschmelze wird auf 90°C abgekühlt und zur Kristallisation stehen gelassen. Ausbeute 95.4%; Smp. 50.5°C.

### Beispiel 2: β-(3-tert-butyl-5-methyl-4-hydroxyphenyl)propionsäurestearylester (Verbindung der Formel I, worin R₁ tert-Butyl ist, R₂ Methyl ist, n = 1 und m = 2 sind, und R₃ für ⁿC₁₈H₃₇ steht)

50 g (0.2 mol) β-(3-tert-butyl-5-methyl-4-hydroxyphenyl)propionsäuremethylester, 53.3 g (0.197 mol) Stearylalkohol und 0.3 g (0.0014 mol) Magnesiumacetat-Tetrahydrat werden in einem Sulfierkolben (5-Halskolben mit flachem Boden) mit KPG-Rührwerk, Thermometer, Wasserabscheider und Stickstoffansatz unter leichtem Stickstoffstrom vorgelegt und aufgeheizt. Ab ca. 100°C spaltet sich Wasser ab. Die Innentemperatur wird auf 185°C erhöht, wobei sich Methanol abspaltet. Nach 4 h bei dieser Temperatur wird auf ca. 80°C abgekühlt und in 200 ml Toluol aufgenommen. Die Lösung wird im Scheidetrichter zwei Mal mit je 200 ml Wasser gewaschen, die organische Phase über 10 g Na₂SO₄ getrocknet, abfiltriert und eingeengt. Das Rohprodukt wird aus Methanol/Wasser (9:1) umkristallisiert. Man erhält 86.8 g (90.1% d.Th.) eines weißen Pulvers (Smp. 61-63°C)

### Beispiel 3: β-(3-tert-butyl-5-methyl-4-hydroxyphenyl)propionsäuretriethylenglykolester (Verbindung der Formel I, worin R₁ tert-Butyl ist, R₂ Methyl ist, n = 2 und m = 2 sind, und R₃ für -(CH₂CH₂O)₂CH₂CH₂-steht)

125.2 g (0.5 mol) β-(3-tert-butyl-5-methyl-4-hydroxyphenyl)propionsäuremethylester, 37.5 g (0.25 mol) Triethylenglykol und 1.5 g (0.0070 mol) Magnesiumacetat-Tetrahydrat werden in einem Sulfierkolben (5-Halskolben mit flachem Boden) mit KPG-Rührwerk, Thermometer, Wasserabscheider und Stickstoffansatz unter leichtem Stickstoffstrom vorgelegt und aufgeheizt. Ab ca. 100°C spaltet sich Wasser ab. Die Innentemperatur wird auf 200°C erhöht, wobei sich Methanol abspaltet. Nach 5 h bei dieser Temperatur legt man einen Unterdruck von 140 mbar an und läßt weitere 2 Stunden weiterreagieren. Anschließend wird auf ca. 80°C abgekühlt und in 200 ml Toluol aufgenommen. Die Lösung wird im Scheidetrichter zwei Mal mit je 200 ml Wasser gewaschen, die organische Phase über 10 g Na₂SO₄ getrocknet, abfiltriert und eingeengt. Das Rohprodukt wird aus Methanol/Wasser (9:1) umkristallisiert. Man erhält 121.5 g (82.9% d.Th.) eines weißen Pulvers (Smp. 61-63°C).

### Beispiel 4: β-(3,5-Di-tert-butyl-4-hydroxyphenyl)propionsäurestearylester (Verbindung der Formel I, worin R₁ und R₂ tert-Butyl sind, n = 1 und m = 2 sind, und R₃ für ⁿC₁₈H₃₇ steht)

258 g (0.88 mol) β-(3,5-Di-tert-butyl-4-hydroxyphenyl)propionsäuremethylester und 185 g (0.68 mol) Stearylalkohol (trocken) werden vorgelegt und bei 80°C und 20 mbar geschmolzen. Wenn alles geschmolzen ist, wird das Vakuum mit Stickstoff gebrochen, und 1 g (0.012 mol) Magnesiumacetat wird zugegeben. Es wird auf 185°C aufgeheizt, binnen einer Stunde auf 3 mbar evakuiert und 2 h bei 185°C gehalten. Danach wird das Vakuum auf unter 1 mbar erhöht und bei 190°C der Überschuß an β-(3,5-Di-tert-butyl-4-hydroxyphenyl)propionsäuremethylester während einer Stunde abdestilliert.
Die Schmelze enthält noch weniger als 0.1% Stearol und 1% β-(3,5-Di-tert-butyl-4-hydroxyphenyl)propionsäuremethylester. Die Reaktionsschmelze wird auf 90°C abgekühlt und zur Kristallisation stehen gelassen. Ausbeute 98% d. Th.; Smp. 50.5°-51°C.

## Patentansprüche

1. Verfahren zur Herstellung von Verbindungen der Formel I 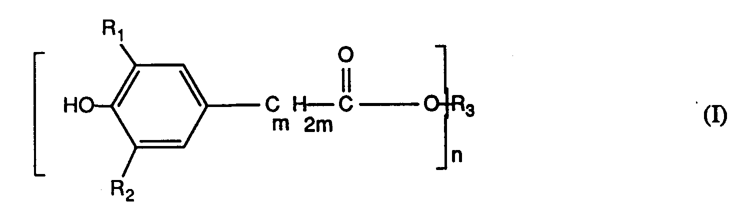
worin R₁ und R₂ unabhängig voneinander C₁-C₈-Alkyl oder Cyclopentyl oder - hexyl sind,
m 0, 1, 2, oder 3 ist,
n 1 oder 2 ist, und
R₃ je nach dem Wert von n C₄-C₂₀-Alkyl, C₅-C₁₂-Cycloalkyl, C₂-C₄-Alkylen oder durch Sauerstoff unterbrochenes C₄-C₁₂-Alkylen ist,
durch Umsetzung einer Verbindung der Formel II 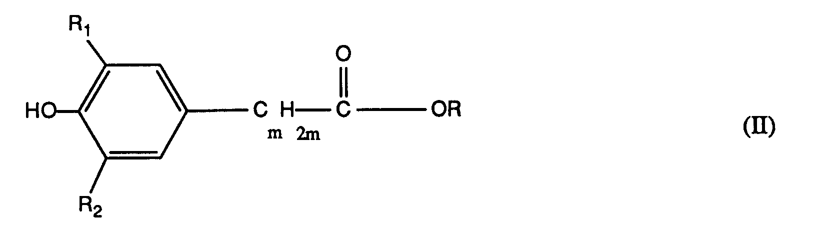
worin R für Methyl oder Ethyl steht,
mit einer Verbindung der Formel III
R₃(OH)ₙ (III)
dadurch gekennzeichnet, daß die Umsetzung in Gegenwart von Magnesiumacetat als Katalysator durchgeführt wird.

2. Verfahren nach Anspruch **1**, wobei m für 2 steht.

3. Verfahren nach Anspruch **1**, wobei R₁ und R₂ C₁-C₄-Alkyl sind.

4. Verfahren nach Anspruch **2**, wobei R₃ C₈-C₂₀-Alkyl oder durch Sauerstoff unterbrochenes C₄-C₁₂-Alkyl ist.

5. Verfahren nach Anspruch **2**, wobei R₁ Methyl oder tert-Butyl und R₂ tert-Butyl darstellen, R₃ n-Octadecyl, 2-Ethylhexyl, i-Octyl oder -(CH₂CH₂O)ₐCH₂CH₂-ist und a für 1 oder 2 steht.

6. Verfahren nach Anspruch **1**, wobei die Umsetzung bei Temperaturen von 120 bis 200°C durchgeführt wird.

7. Verfahren nach Anspruch **1**, wobei der Druck während der Umsetzung 1 bis 1000 mbar beträgt.

8. Verfahren nach Anspruch **1**, wobei der Druck während der Umsetzung 1 bis 200 mbar beträgt.

9. Verfahren nach Anspruch **1**, wobei nach der Reaktion das Produkt ungereinigt mit physikalischen Methoden in eine verwendungsfähige Handelsform gebracht wird.

10. Verwendung von Magnesiumacetat als Katalysator bei der Herstellung von Verbindungen der Formel I gemäß Anspruch **1** durch Umsetzung von Verbindungen der Formel II gemäß Anspruch **1** mit Verbindungen der Formel III gemäß Anspruch **1**.

## Claims

1. A process for the preparation of a compound of formula I 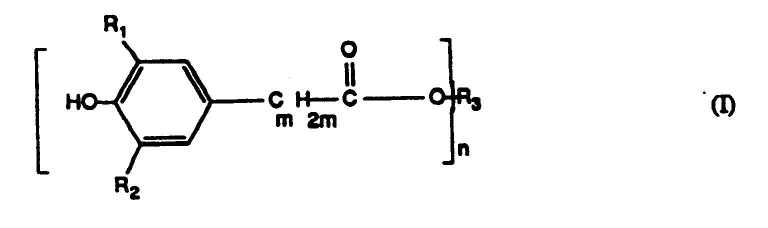
wherein R₁ and R₂ are each independently of the other C₁-C₈alkyl or cyclopentyl or cyclohexyl,
m is 0, 1, 2 or 3,
n is 1 or 2, and
R₃, depending on the value of n, is C₄-C₂₀alkyl, C₅-C₁₂cycloalkyl, C₂-C₄alkylene, or C₄-C₁₂alkylene which is interrupted by oxygen,
by reaction of a compound of formula II 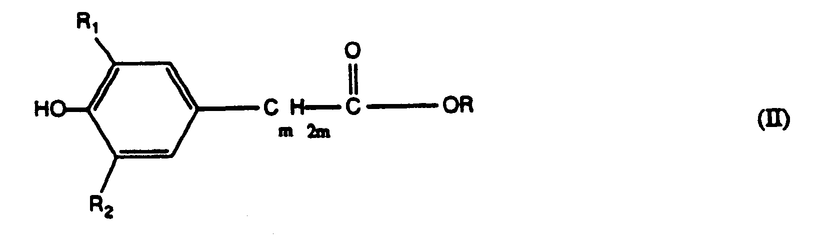
wherein R is methyl or ethyl
with a compound of formula III
R₃(OH)ₙ (III)
which reaction is carried out in the presence of magnesium acetate as catalyst.

2. A process according to claim 1, wherein m is 2.

3. A process according to claim 1, wherein R₁ and R₂ are C₁-C₄alkyl.

4. A process according to claim 2, wherein R₃ is C₈-C₂₀alkyl, or C₄-C₁₂alkyl which is interrupted by oxygen.

5. A process according to claim 2, wherein R₁ is methyl or tert-butyl and R₂ is tert-butyl, R₃ is n-octadecyl, 2-ethylhexyl, isooctyl or -(CH₂CH₂O)ₐCH₂CH₂-, and a is 1 or 2.

6. A process according to claim 1, wherein the reaction is carried out at temperatures from 120 to 200°C.

7. A process according to claim 1, wherein the pressure during the reaction is from 1 to 1000 mbar.

8. A process according to claim 1, wherein the pressure during the reaction is from 1 to 200 mbar.

9. A process according to claim 1, wherein after the reaction the product is brought without purification into a useful commercial form by physical methods.

10. Use of magnesium acetate as catalyst in the preparation of a compound of formula I according to claim 1 by reacting a compound of formula II according to claim 1 with a compound of formula III according to claim 1.

## Revendications

1. Procédé de préparation de composés de formule I 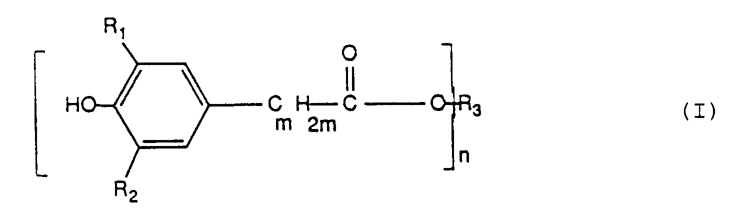 dans laquelle
R₁ et R₂ représentent, indépendamment l'un de l'autre, un groupe alkyle en C₁-C₈ ou un groupe cyclopentyle ou cyclohexyle,
m vaut 0, 1, 2 ou 3,
n vaut 1 ou 2, et
R₃ représente, en fonction de la valeur de n, des groupes alkyle en C₄-C₂₀, cycloalkyle en C₅-C₁₂, alkylène en C₂-C₄ ou alkylène en C₄-C₁₂ interrompu par un atome d'oxygène,
par réaction d'un composé de formule II 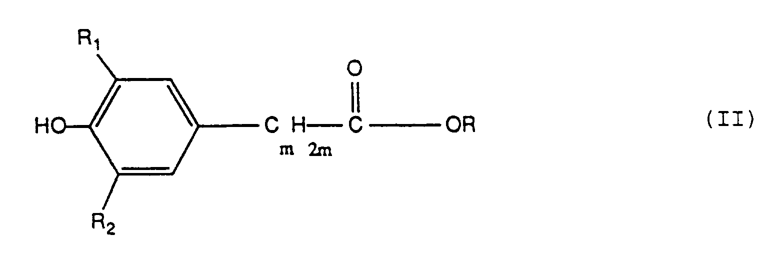 dans laquelle
R représente un groupe méthyle ou éthyle,
avec un composé de formule III
R₃(OH)ₙ (III)
caractérisé en ce que l'on met en oeuvre la réaction en présence d'acétate de magnésium comme catalyseur.

2. Procédé selon la revendication 1, m ayant la valeur de 2.

3. Procédé selon la revendication 1, R₁ et R₂ représentant un groupe alkyle en C₁-C₄.

4. Procédé selon la revendication 2, R₃ étant un groupe alkyle en C₃-C₈ ou un groupe alkyle en C₄-C₁₂ interrompu par un atome d'oxygène.

5. Procédé selon la revendication 2, R₁ représentant un groupe méthyle ou tert-butyle et R₂ représente un groupe tert-butyle, R₃ représente un groupe n-octadécyle, 2-ethylhexyle, i-octyle ou -(CH₂CH₂O)ₐCH₂CH₂- et a valant 1 ou 2.

6. Procédé selon la revendication 1, la réaction étant mise en oeuvre à une température de 120 à 200 °C.

7. Procédé selon la revendication 1, la pression au cours de la réaction étant de 1 à 1000 mbar.

8. Procédé selon la revendication 1, la pression au cours de la réaction étant de 1 à 200 mbar.

9. Procédé selon la revendication 1, le produit de réaction non purifié pouvant être mis en forme commerciale utilisable, au moyen de méthodes physiques.

10. Utilisation d'acétate de magnésium en tant que catalyseur pour la préparation de composés de formule I, selon la revendication 1, par réaction de composés de formule II selon la revendication 1 avec des composés de formule III selon la revendication 1.
